Europäisches Patentamt

**European Patent Office**

Office européen des brevets

(11) Publication number:

# 0 286 415
## A2

⑫ # EUROPEAN PATENT APPLICATION

㉑ Application number: **88303138.7**

㉒ Date of filing: **08.04.88**

(51) Int. Cl.⁴: **A 61 B 17/32**

㉚ Priority: **09.04.87 GB 8708481**

㊸ Date of publication of application:
**12.10.88 Bulletin 88/41**

㊽ Designated Contracting States:
**AT BE CH DE ES FR GB IT LI LU NL**

⑪ Applicant: **Wickham, John Ewart Alfred**
**27 Welbeck Street**
**London, W.1. (GB)**

**Coptcoat, Malcolm John**
**31 Famet Avenue**
**Purley Surrey (GB)**

**Carter, Simon**
**66 Archer House Vicarage Crescent**
**London, SW11 (GB)**

⑫ Inventor: **Wickham, John Ewart Alfred**
**27 Welbeck street**
**London W1 (GB)**

**Coptcoat, Malcolm John**
**31 Famet Avenue**
**Purley, Surrey (GB)**

⑭ Representative: **Thiemann, Peter Albert William et al**
**LLOYD WISE, TREGEAR & CO. Norman House 105-109**
**Strand**
**London WC2R 0AE (GB)**

(54) **Tissue disintegrator.**

(57) A tissue disintegrator is described and is adapted for use with an endoscope for the surgical removal of tissue. The disintegrator comprises a cutter blade mounted at the end of an elongated drive shaft the other end of which is connected to means for rotating the drive shaft. The drive shaft is supported for rotation in a hollow sheath and a suction path parallel to the drive shaft leads from the cutter blade to an attachment for connection to a source of suction, so that disintegrated tissue can be removed by suction from the region in which the cutter blade operates. In order to prevent the cutter blade from binding on the tissue, the region in which the cutter blade operates may be flushed with a physiologically acceptable irrigation liquid or a reciprocating motion may be imparted to the drive shaft.

FIG.I.

EP 0 286 415 A2

## Description

## TISSUE DISINTEGRATOR

This invention relates to a tissue disintegrator for the surgical removal of tissue.

The techniques of endoscopic surgery have brought great advances in patient care. The avoidance of large muscle-cutting incisions has meant that patients recover more quickly from operations, have less pain and trauma and return to normal activities earlier than with earlier techniques. However, a problem with endoscopic surgery is that tissue having a bulk larger than the diameter of the access site, which may be a natural orifice or a percutaneous access site cannot be removed. Several techniques have been proposed to remove such tissue but these are, at best, slow, tedious and piecemeal techniques.

It is an object of the invention to provide a tissue disintegrator, the use of which will enable tissue having a bulk larger than the diameter of an access site to be removed rapid, easily and with a minimum of trauma.

According to the present invention there is provided a tissue disintegrator comprising a cutter blade and means for rotating the latter, characterised in that the cutter blade is mounted at one end of an elongated drive shaft, the other end of which is connected to the means for rotating the drive shaft, which is supported for rotation in an elongated hollow sheath, and in that the disintegrator is provided with an attachment for connection to a source of suction and with means defining a suction path parallel to said drive shaft and leading from said cutter blade to said attachment, whereby disintegrated tissue can be removed by suction from the region in which the cutter blade operates, the disintegrator being adapted for use with an endoscope.

In one embodiment of the invention means may be provided for imparting not only a rotary motion but also a reciprocating motion to the drive shaft and thus to the cutter blade. The reciprocating motion can be imparted to the drive shaft by means of a cam mounted on a two-part spindle, the two parts of which are keyed to each other. In this embodiment, it may be desirable to make the drive shaft hollow so that it defines the suction path. In this case the other end of the drive shaft is mounted in a housing which is formed with a chamber into which the attachment for connection to a source of suction leads, and the drive shaft is formed with at least one aperture in the chamber. If desired, a further shaft surrounding the sheath supporting the drive shaft or a tube running alongside the supporting sheath can be provided for supplying an irrigation liquid to the cutting site or region in which the cutter blade operates, the irrigation liquid together with the disintegrated tissue being removed along the suction path.

In another embodiment of the invention, simultaneous reciprocating motion is not imparted to the drive shaft but the cutting site is well irrigated. This irrigation and a high speed of rotation for the cutter blade serve to prevent the cutter blade from binding on the tissue. In this embodiment, the drive shaft and its support sheath are surrounded by an outer hollow sheath which is longitudinally divided to form the suction path and a path for leading an irrigation liquid to the region in which the cutter blade operates, the irrigation liquid and disintegrated tissue being removed along the suction path. Because the cutting site is washed by the irrigation liquid, it is possible for the surgeon to obtain a clear view of the cutting side through the endoscope with which the disintegrator is used.

The drive shaft and its support sheath constitute a probe which is, in use, passed down an endoscope. While the drive shaft is preferably closely surrounded by a support sheath, in certain circumstances the wall of the endoscope can act as the support sheath and in this case, either the outside of the drive shaft or the inside of the endoscope may have annular bearing pads to support the drive shaft.

In order to enable the invention to be more readily understood, reference will now be made to the accompanying drawings, which illustrate diagrammatically and by way of example an embodiment thereof, and in which:-

Figure 1 is a longitudinal section through a tissue disintegrator,

Figure 2 is a series of sectional and corresponding plan views of a variety of cutters for the disintegrator shown in Figure 1,

Figure 3 is a longitudinal section through another tissue disintegrator,

Figure 4 is a section along the line IV-IV in Figure 3, and

Figure 5A to 5C shown plan and associated elevational views of different cutter blades.

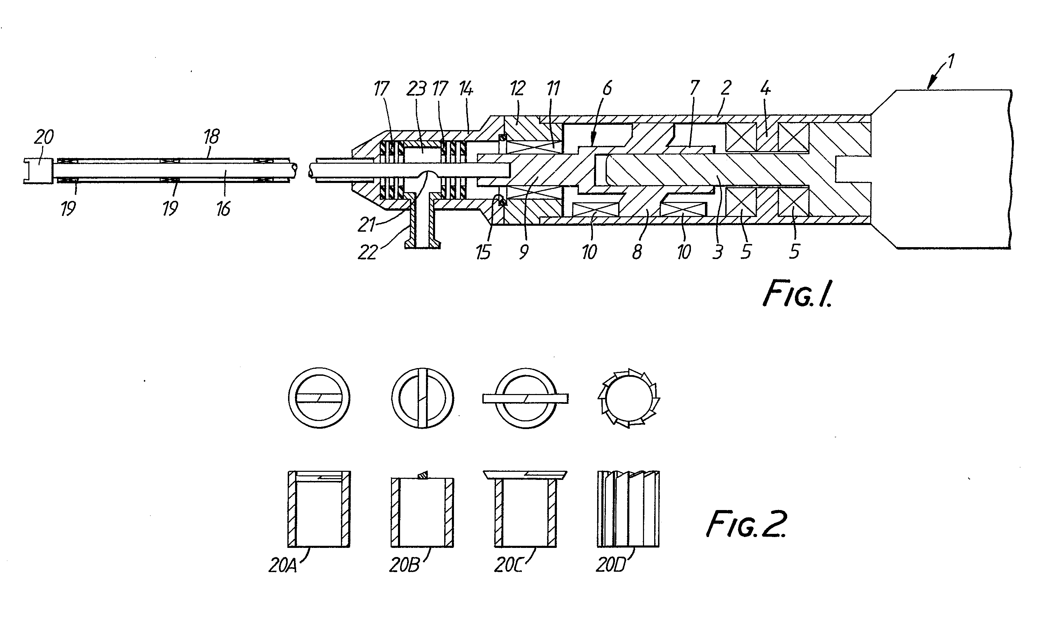

Referring now to Figure 1 there is shown a tissue disintegrator comprising a housing 1 which contains an electric motor of conventional construction. The housing 1 is intended to be held on the hand of an operator and the housing and motor will not be described further. Attached to the housing 1 is a hollow tube 2 which contains a spindle 3 adapted to be rotated by the motor. The tube 2 has an internal annular collar 4 for supporting bearings 5 for the spindle 3. A driving member 6, in the form of a hollow tube 7 carrying a cam 8 and terminating in a forward extension 9, is mounted on the end of the spindle 3 and is keyed thereto so as to be capable of rotating with the spindle and to be capable of movement in the axial direction relative to the spindle. The cam 8 is held between two fixed bearings 10 mounted on the interior of the tube 2. The cam 8 is shaped so that as the driving member 6 rotates, a reciprocating motion in the axial direction is imparted to it by the engagement of the cam with the bearings 10. The forward extension 9 of the driving member 6 is supported by an annular bearing 11 mounted in an end member 12 secured as by welding or otherwise to the hollow tube 2.

A hollow nose-piece 14 is secured to the end member 12 with the interposition of an 0-ring seal 15 and a hollow drive shaft 16 is mounted fast in the leading end of the forward extension 9, the hollow drive shaft passing through the nose-piece and being mounted in seals 17. A hollow sheath 18 is secured in the leading end of the nose-piece 14 and the drive shaft 16 passes through the sheath and is supported therein by bearing pads 19. The drive shaft 16 carries at its end a cylindrical cutter 20 and is formed with an aperture 21 located in the nose-piece 14. A suction outlet pipe 22 passes through the wall of the nose-piece 14 into a chamber 23 which is formed between the seals 17 and in which the aperture 21 is located.

Figure 2 shows various forms which the cutter 20 may take. The cutter 20A has a recessed cutting blade, the cutter 20B has a projecting cutting blade, the cutter 20C has a projecting cutting blade of greater diameter than the cylindrical body of the cutter and the cutter 20D has a serrated or saw-tooth rim and body. The cutters are made interchangeable so that an appropriate cutter can be secured to the end of the hollow drive shaft 16 in a manner not shown.

When the electric motor in the housing 1 is switched on, the spindle 3 is rotated and as it is keyed to the driving member 6, the latter also rotates. Due to the shape of the cam 8, a reciprocating motion is also imparted to the driving member 6 and thus to the drive shaft and cutter which also rotate.

In the use of the tissue disintegrator just described, an endoscope is inserted into the patient, either through a natural orifice or through a percutaneous access site, up to the tissue to be removed. The drive shaft 16 and its sheath 18 are then introduced through the endoscope and the motor is switched on, whereupon the cutter acts on the tissue to be removed. The reciprocating motion of the cutter prevents the cutter from binding on the tissue or being brought to a halt by reason of the torque in the drive shaft and an efficient disintegration of the tissue occurs. Suction is applied to the outlet pipe 22 and the disintegrated tissue is extracted by suction through the hollow drive shaft into the chamber 23 from where it flows out through the pipe 22.

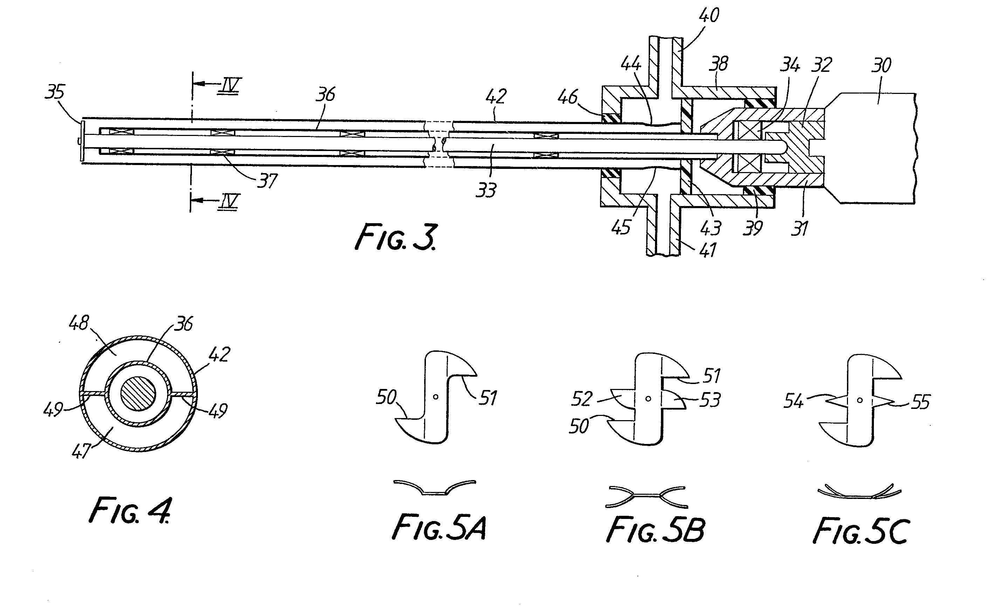

Referring now to Figures 3 and 4, there is shown another embodiment of the tissue disintegrator but in which an irrigation liquid is supplied to the cutting site and no reciprocating motion is applied to the spindle or drive shaft. The tissue disintegrator shown in Figure 2 comprises a housing 30 which contains an electric motor of conventional construction and is intended to be held in the hand of an operator. A hollow nose-piece 31 is secured to the housing 30 and contains a spindle 32 adapted to be rotated by the electric motor in the housing 30. A drive shaft 33, in the form of a solid rod, is mounted fast at one end in the end of the spindle 32, the drive shaft passing through an aperture in the nose-piece 31 and being supported within the nose-piece by bearings 34. A cutter blade 35 is mounted on the free end of the drive shaft 33 and is fixed in such a manner that it can be readily interchanged with other cutter blades.

The drive shaft 33 is surrounded by a hollow sheath 36, one end of which is secured fast in the end of the nose-piece 31 and the other end of which terminates near the cutter blade 35. The drive shaft 33 is supported in the hollow sheath 36 by bearing pads 37.

An open-ended housing 38 is placed over the end of the nose-piece 31 with the interposition of a seal 39. The housing 38 has an inlet 40 for connection to a source of irrigation liquid, and an outlet 41 for connection to a source of suction. An outer hollow sheath 42 surrounds the sheath 36 and has one end secured in a sealing disc 43 mounted in the housing and aperture to receive the hollow sheath 36 with a sealing fit. Adjacent the sealing disc 43, the sheath 36 is formed with an inlet aperture 44 and an outlet aperture 45, and is secured in the end of the housing 38 by means of a seal 46. The annular space between the hollow sheaths 36 and 42 is divided into two channels 47 and 48 by wall members 49 which, while they are shown in Figure 4 as being integral with the hollow sheaths 36 and 42 may take the form of separate inserts or be integral with one or other only of the sheaths.

Figures 5A, 5B and 5C show various forms which the cutter blade 35 may take. The blade shown in Figure 5A has cutting edges 50 and 51 and, end on, is of gull wing shape. The blade shown in Figure 5B also has cutting edges 52 and 53 and the blade shown in Figure 5C is similar to that shown in Figures 5B but has intermediate cutting blades 54 and 55 of different shape, the intermediate cutting blades 52 and 53 being bent in the opposite direction to the edges 51 and 52.

The operation of the disintegrator shown in Figures 3 to 5 is similar to that of the disintegrator shown in Figures 1 and 2 except that no reciprocating motion is imparted to the drive shaft 33. When the electric motor in the housing 30 is switched on, the spindle 32 is rotated and directly rotates the drive shaft 33. The disintegrator is introduced into a patient through an endoscope and the motor is switched on. At the same time or earlier, an irrigation liquid is introduced to the operation site through the inlet 40, the inlet aperture 44, and the channel 48. The irrigation liquid flushes the operation site and is sucked out through the channel 47, the outlet aperture 45 and the outlet 41. Water or saline is a suitable irrigation liquid but other physiologically compatible liquids or solutions may be used if desired.

By appropriate choice of the speed of rotation of the drive shaft 33 and thus the cutting blade 35 and by an appropriate rate of flushing of the operation site with the irrigation liquid it can be ensured that the cutter does not bind on the tissue and is not brought to a halt by torque in the drive shaft.

It will be appreciated that the size of the present disintegrator is determined by the size of the endoscope with which it is to be used and the type of operation for which it is intended to be used. In general, the diameter of the sheath 18 or 36 will be in the region of 5.0 mm and the hollow sheath 42 will be not greater than 10.0 mm in diameter. Sizes larger than these are not compatible with the surgical techniques involved.

The speed of rotation and the geometry of the cutting blade 20 or 35 are important factors governing the rate of tissue extraction and the efficiency of operation of the disintegrator. The speed of rotation of the blade may be as high as 40,000 r.p.m. and is preferably not less than about 10,000 r.p.m. At these speeds it is possible to ensure an effective speed of the blade relative to the tissue being cut so as to ensure good results in terms of tissue extraction rates.

With the disintegrator described above, the cutting blades are made interchangeable so that it is possible to select an appropriate cutting blade for the different kinds of tissue which may have to be disintegrated and removed.

In the disintegrator described above, it is a feature that the drive shaft 16 or 33 rotates within a hollow sheath 18 or 36. The function of this hollow sheath is not only to give support to the drive shaft as it rotates at high speed but also to prevent fibrous tissue from being caught around the drive shaft and causing the latter to show down, thereby possibly tearing rather than cutting tissue, and causing the drive shaft to rotate off centre.

It will be appreciated that many modifications of the disintegrators described above are possible. For example, the disintegrator shown in Figures 1 and 2 could be provided with an outer sheath, similar to the sheath 42 in Figures 3 and 4 so that the cutting site can be irrigated with a suitable liquid, or the disintegrator shown in Figures 3 and 4 can be provided with means for imparting a reciprocating motion to the cutter.

The disintegrator is preferably made of stainless steel but can be made of other suitable materials which are compatible with body fluids and capable of being sterilized.

Bladder obstruction caused by an enlarged prostrate gland is a very common condition in middle aged and elderly men and the removal of the enlarged gland is a slow, tedious and piecemeal operation at present. However, with either of the disintegrators described above it is possible to rebore the bladder outlet in a matter of minutes.

The disintegrator can also be used for bulk tissue removal in minimally invasive operations, such as the removal of a kidney, gall bladder fibroids and other abdominal and pelvic tumours and organs through natural orifices or small percutaneous puncture sites.

Ultrasonic and other scanning techniques exist for determining the extent of tumour or other unwanted tissue, and in a further development of the present invention, the present tissue disintegrator is fitted to a robot arm the movement and operation of which is controlled by a computer. The tissue to be removed is scanned electronically or otherwise and the resulting information is fed to the computer which is programmed to set the parameters of operation of the robot arm and guide the latter so that the tissue is removed automatically. By this means it is possible to reduce considerably the time taken for operations as the surgeon does not have to keep removing the disintegrator from the endoscope and visually monitoring the operation.

## Claims

1. A tissue disintegrator comprising a cutter blade and means for rotating the latter, characterised in that the cutter blade is mounted at one end of an elongated drive shaft, the other end of which is connected to the means for rotating the drive shaft, which is supported for rotation in an elongated hollow sheath, and in that the disintegrator is provided with an attachment for connection to a source of suction and with means defining a suction path parallel to said drive shaft and leading from said cutter blade to said attachment, whereby disintegrated tissue can be removed by suction from the region in which the cutter blade operates, the disintegrator being adapted for use with an endoscope.

2. A tissue disintegrator as claimed in Claim 1, wherein means is provided for imparting a reciprocating motion to the drive shaft and thus to the cutter blade.

3. A tissue disintegrator as claimed in Claim 1 or 2, wherein the drive shaft is hollow and defines said suction path.

4. A tissue disintegrator as claimed in any one of Claims 1 to 3, wherein the disintegrator is provided with means for leading an irrigation liquid to the region in which the cutter blade operates.

5. A tissue disintegrator as claimed in Claim 1, wherein the hollow supporting sheath for the drive shaft is surrounded by an outer hollow sheath which is longitudinally divided to form said suction path and a path for leading an irrigation liquid to the region in which the cutter blade operates.

6. A tissue disintegrator as claimed in any preceding claim, wherein the cutter blade is detachably mounted.

*FIG. 1.*

*FIG. 2.*

FIG. 3.

FIG. 4.

FIG. 5A

FIG. 5B

FIG. 5C